# EUROPÄISCHE PATENTSCHRIFT

(11) **EP 0 713 869 B1**
(45) Veröffentlichungstag und Bekanntmachung des Hinweises auf die Patenterteilung: **28.04.1999**
(21) Anmeldenummer: 95118473.8
(22) Anmeldetag: 23.11.1995
(51) Int. Cl.: C07D 213/61, C07D 213/64, C07D 401/04, C07D 405/04, A61K 31/44, A61K 31/50

(54) **Di- und trisubstituierte Pyridine und Verfahren zu ihrer Herstellung**
Di- and trisubstituted pyridine derivatives and a process for their preparation
Derivés de pyridine di et trisubstitués et leur préparation

(30) Priorität: 25.11.1994 CH 3537/94; 25.11.1994 CH 3538/94
(43) Veröffentlichungstag der Anmeldung: 29.05.1996
(73) Patentinhaber: LONZA AG, CH-3945 Gampel/Wallis (CH)
(72) Erfinder: Kiener, Andreas, Dr., Visp (Kanton Wallis) (CH); Roduit, Jean-Paul, Dr., Grône (Kanton Wallis) (CH); Wellig, Alain, Ried-Mörel (Kanton Wallis) (CH)
(74) Vertreter: Weinhold, Peter, Dr.

(56) Entgegenhaltungen:
- EP-A- 0 556 465
- WO-A-95/06032
- DE-A- 2 634 188
- US-A- 5 089 509

## Beschreibung

Die vorliegende Erfindung betrifft neue 2,5-di- und 2,3,5-trisubstituierte Pyridine sowie Verfahren zu ihrer Herstellung aus Nicotin.

2,5-Disubstituierte (bzw. 3,6-disubstituierte bei alternativer Bezifferung) Pyridine wie beispielsweise 6-Hydroxynicotinsäure oder 6-Chlornicotinsäure sind wertvolle chemische Zwischenprodukte, beispielsweise zur Herstellung von Retinoid-Analogen (US 5 089 509) oder Renin-Inhibitoren (US 5 098 924).
Die bisher im technischen Massstab erhältlichen 2,5-disubstituierten Pyridine sind Verbindungen, die wie die 6-Hydroxynicotinsäure in der Regel in der Position 5 (bzw. 3) nur einen C₁-Rest tragen.
Für die Herstellung von Verbindungen mit längeren Kohlenstoffketten muss dieser C₁-Rest durch herkömmliche Synthesemethoden unter C-C-Verknüpfung verlängert werden (US 5 089 509, Spalte 13). Da diese Methode umständlich und teuer ist, war es wünschenswert, Verbindungen mit längeren Seitenketten und für weitere Syntheseschritte verwendbaren funktionellen Gruppen auf einfacherem Weg zu erhalten. Weiterhin war es wünschenswert, Verbindungen dieser Klasse zugänglich zu machen, die als zusätzlichen Substituenten in der Position 3 des Pyridinrings (bei Bezifferung als 2-Pyridon) ein Chloratom tragen.
Aufgabe der vorliegenden Erfindung war daher, neue 2,5-disubstituierte Pyridine mit längeren Seitenketten in der Position 5 und gegebenenfalls einem Chloratom in der Position 3 des Pyridinringes bereitzustellen und einen einfachen Weg, ausgehend von gut zugänglichen Edukten, zu diesen neuen Verbindungen aufzuzeigen.
Erfindungsgemäss wird diese Aufgabe durch die Verbindungen nach Patentanspruch 1 und die Herstellungsverfahren nach den Patentansprüchen 2 5, 9, 13, 14 und 15 gelöst.

Es wurde gefunden, dass aus dem in grossen Mengen verfügbaren nachwachsenden Rohstoff Nicotin in technisch verwertbarem Massstab durch mikrobiologische Oxidation mit Mikroorganismen der Gattungen *Pseudomonas* und *Variovorax* das an sich bekannte 5-Succinoyl-2-pyridon (4-(1,6-Dihydro-6-oxopyridin-3-yl)-4-oxobuttersäure) gewonnen und auf chemischem Weg in neue 2,5-disubstituierte Pyridine mit einer C₄-Seitenkette oder einem entsprechenden heterocyclischen Rest in der Position 5 des Pyridinringes übergeführt werden kann.
Die hier und im folgenden genannten 2-Pyridone sind bekanntlich tautomeriefähige Verbindungen und können auch in der 2-Hydroxypyridin-Form oder als Gemisch der beiden tautomeren Formen vorliegen. Wenn im folgenden jeweils nur eine der beiden möglichen Formen genannt oder als Strukturformel abgebildet ist, so sind damit immer beide Formen gemeint, und zwar unabhängig davon, in welcher Form die jeweilige Verbindung tatsächlich vorliegt.

Dass Nicotin auf mikrobiologischem Weg zu 5-Succinoyl-2-pyridon oxidiert werden kann, ist an sich bekannt, beispielsweise aus der DE-OS 26 34 188.
Es wurde jedoch gefunden, dass bei Verwendung bestimmter Stämme von *Pseudomonas* oder *Variovorax* und insbesondere bei Einhaltung relativ hoher Nicotinkonzentrationen besonders hohe Ausbeuten und Produktkonzentrationen erhalten werden.

Die vorzugsweise verwendeten Mikoorganismen wurden mittels üblicher mikrobiologischer Techniken aus Klärschlamm bzw. Erde isoliert und mit Nicotin als Wachstumssubstrat selektioniert.
Sie wurden bei der DSM-Deutsche Sammlung von Mikroorganismen und Zellkulturen GmbH, Mascheroder Weg 1b, D-38124 Braunschweig hinterlegt.
Die Einzelheiten der Isolierung und Selektionierung sind im Beispiel 1 beschrieben.

Die Eigenschaften der Stämme *P. putida* DSM 8231, *P. fluorescens* DSM 8235, *P. putida* DSM 8236 und *V. paradoxus* DSM 8244 sind in den folgenden Übersichten zusammengefasst.

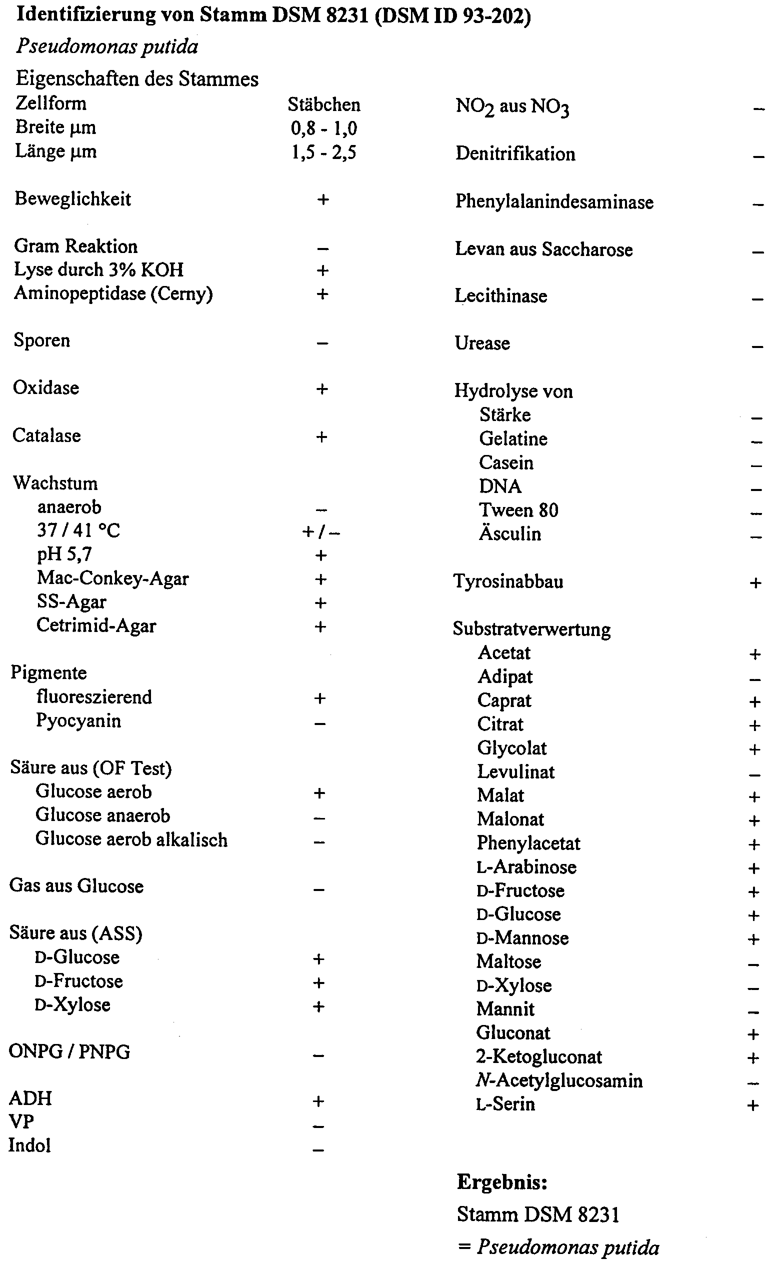

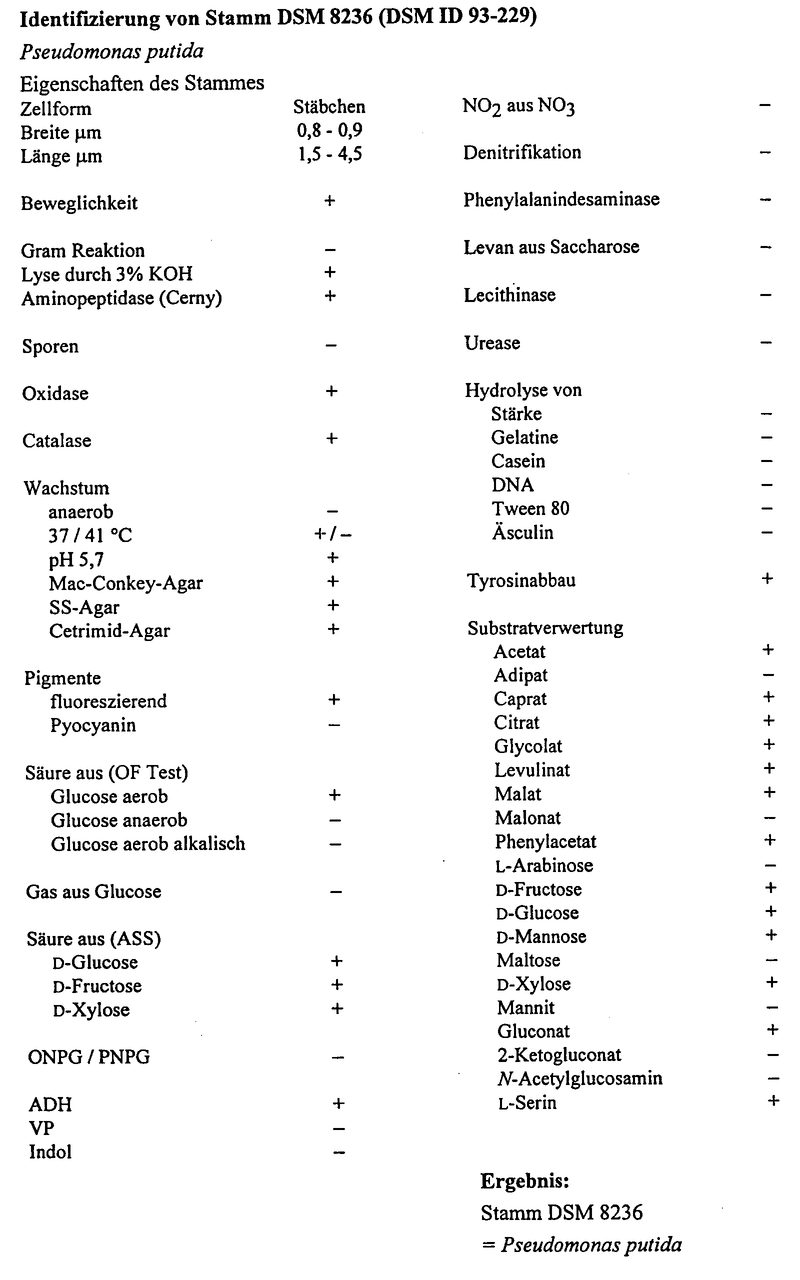

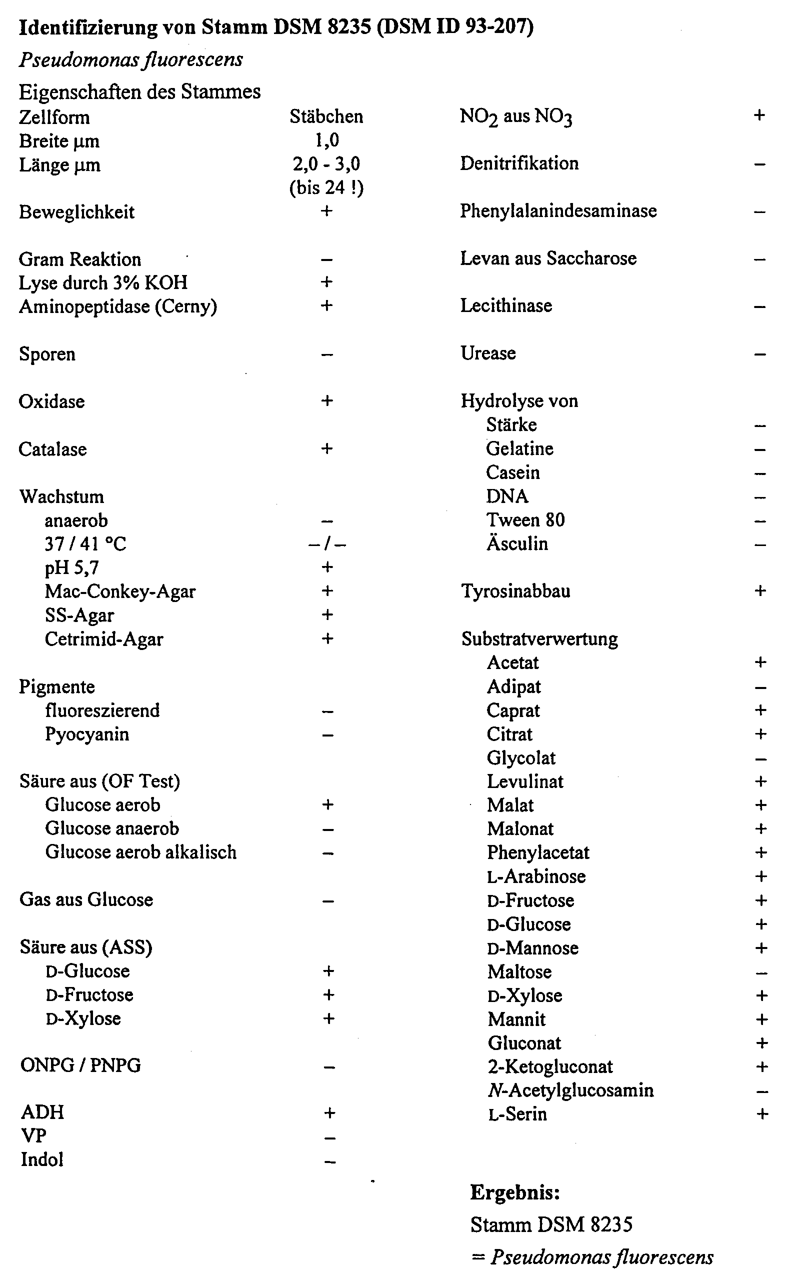

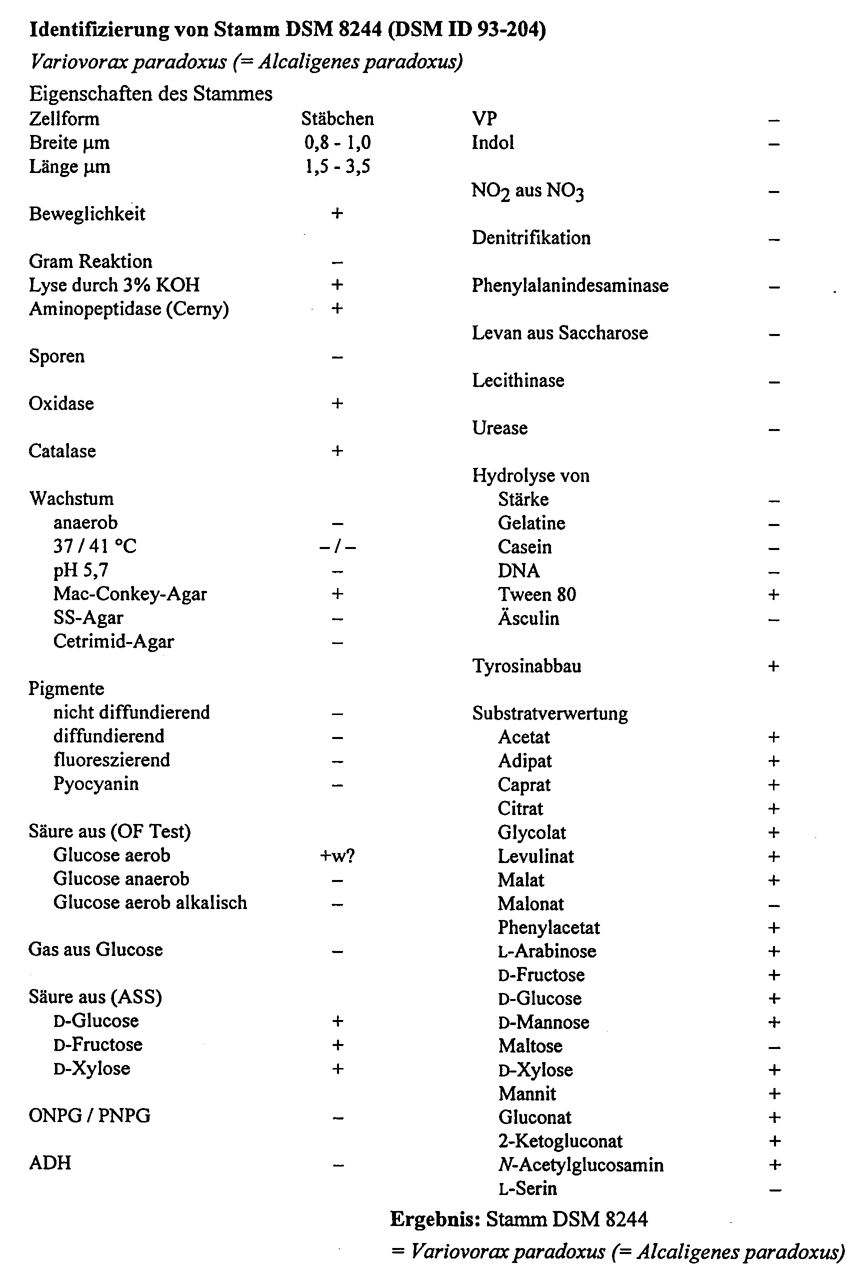

Die Biotransformation von Nicotin zu 5-Succinoyl-2-pyridon erfolgt auf übliche Weise unter aeroben Bedingungen durch Anzucht eines Inokulums, mit dem ein Fermenter angeimpft wird. Vorteilhaft wird ein Teil des Substrats (Nicotin) vorgelegt und der Rest kontinuierlich zugeführt. Die Nikotinkonzentration während der Transformation beträgt zweckmässig 1 bis 50 g/l, vorzugsweise 5 bis 20 g/l. Der pH-Wert liegt während der Transformation zweckmässig im Bereich von 4 bis 9, vorzugsweise etwa bei 7. Er wird vorzugsweise durch geregelten Zusatz von Säure und/oder Base konstant gehalten. Die Temperatur liegt während der Transformation zweckmässig zwischen 20 und 50 °C, vorzugsweise bei 25 bis 45 °C. Das Produkt kann aus dem zellfreien Kulturüberstand auf übliche Weise isoliert werden, beispielsweise durch Ausfällen mit Säure und Filtration.

Vorzugsweise wird die Biotransformation mit den bereits genannten Stämmen *P. putida* DSM 8231, *P. fluorescens* DSM 8235, *P. putida* DSM 8236 *V. paradoxus* DSM 8244 oder *P. sp.* DSM 8653 durchgeführt.

Die erfindungsgemässen Verbindungen leiten sich von 5-Succinoyl-2-pyridon ab und haben die allgemeine Strukturformel 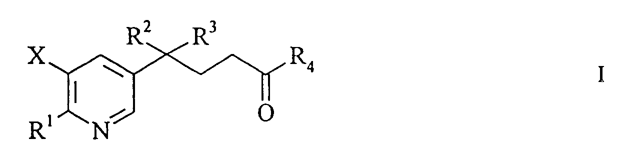
R¹ steht hierbei für Hydroxy oder Chlor. Wie bereits erwähnt, können die Verbindungen mit
R¹= Hydroxy auch in der 2-Pyridon-Form vorliegen

Unter diese allgemeine Formel fallen folgende Gruppen von Verbindungen:
a): X ist Wasserstoff oder Chlor, R² und R³ zusammen sind =O und R⁴ ist eine ―OR⁵-Gruppe mit R⁵ = Wasserstoff, C₁―C₄-Alkyl oder Benzyl. Diesem Fall entsprechen die in Position 2 (bzw. 6) und gegebenenfalls 3 (bzw. 5) des Pyridinrings substituierten 4-(Pyridin-3-yl)-4-oxobuttersäuren und deren C₁―C₄-Alkyl- und Benzylester, wobei das 5-Succinoyl-2-pyridon mit R¹ = R⁴ = ―OH ausgenommen ist.
b): X ist Wasserstoff und R², R³ und R⁴ zusammen bilden eine =N-NH―Gruppe, so dass sich mit der C₄-Seitenkette zusammen ein Tetrahydropyridazinring ergibt.
c): X ist Wasserstoff, R² ist Wasserstoff und R³ und R⁴ zusammen sind ―O―. Diesem Fall entsprechen die Lactone der in Position 2 (bzw. 6) des Pyridinrings substituierten 4-(Pyridin-3-yl)-4-hydroxybuttersäuren.
d): X ist Wasserstoff, R² Wasserstoff, R³ Hydroxy und R⁴ Amino oder Hydroxy. Diesem Fall entsprechen die in Position 2 (bzw. 6) des Pyridinrings substituierten 4-(Pyridin-3-yl)-4-hydroxybuttersäuren und ihre Amide.

Die neuen Verbindungen der ersten Gruppe mit X = Wasserstoff können durch Veresterung des wie oben beschrieben aus Nicotin erhaltenen 5-Succinoyl-2-pyridons mit C₁―C₄-Alkanolen oder Benzylalkohol zum entsprechenden C₄―C₄-Alkyl- oder Benzylester der Formel 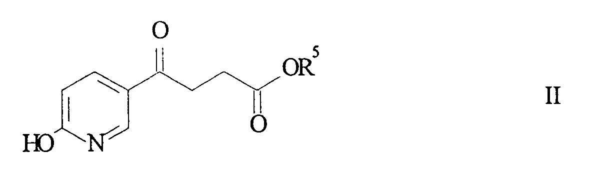 worin R⁵ C₁―C₄-Alkyl oder Benzyl ist, und gegebenenfalls Austausch der Hydroxygruppe gegen Chlor erhalten werden. Falls der Austausch der Hydroxygruppe gegen Chlor durchgeführt wird, wird als Reagens vorzugsweise Phosphoroxychlorid eingesetzt.
Die Herstellung des 2-Chlor-5-succinoylpyridins (R¹ = Cl, R² + R³ = =O, R⁴ = OH) kann beispielsweise durch Hydrolyse eines entsprechenden Alkylesters oder Benzylesters erfolgen.

Die Verbindungen der zweiten Gruppe mit Tetrahydropyridazinring (R² + R³ + R⁴ = =N―NH―) werden erfindungsgemäss hergestellt, indem Nicotin zunächst in der oben beschriebenen Weise mikrobiologisch zu 5-Succinoyl-2-pyridon oxidiert, anschliessend auf ebenfalls bereits beschriebene Weise zum Alkylester (II) verestert, gegebenenfalls durch Austausch der Hydroxygruppe gegen Chlor in die entsprechende Chlorverbindung übergeführt und schliesslich mit Hydrazin zum Pyridazinonderivat der Formel 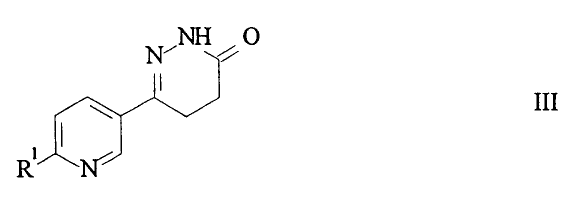 cyclisiert wird.
Für die Cyclisierung wird das Hydrazin vorteilhaft als Hydrazinhydrat eingesetzt. Es ist selbstverständlich auch möglich, wasserfreies Hydrazin zu verwenden.
Die Umsetzung-mit Hydrazin(hydrat) wird vorzugsweise in einem polaren Lösungsmittel durchgeführt, beispielsweise in Methanol oder Ethanol.
Die Reaktionstemperatur beträgt dabei zweckmässig 0 bis 100°C.

Das Lacton der 4-(6-Chlorpyridin-3-yl)-4-hydroxybuttersäure wird erfindungsgemäss hergestellt, indem Nicotin zunächst in der oben beschriebenen Weise mikrobiologisch zu 5-Succinoyl-2-pyridon oxidiert, anschliessend auf ebenfalls bereits beschriebene Weise zum Alkylester (II) oder Benzylester verestert, durch Austausch der Hydroxygruppe in die entsprechende Chlorverbindung übergeführt und anschliessend zum Lacton der Formel 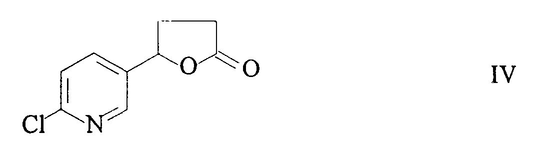 reduziert und cyclisiert wird.
Die Reduktion und Cyclisierung des Esters zum Lacton wird vorzugsweise mit einem Reduktionsmittel aus der Gruppe der komplexen Borhydride durchgeführt. Besonders bevorzugt als Reduktionsmittel ist Natriumborhydrid.
Zur Vervollständigung der Lactonbildung wird das Reaktionsgemisch während oder nach der Reduktion vorteilhaft erwärmt.
Die 4-(6-Chlorpyridin-3-yl)-4-hydroxybuttersäure selbst sowie ihr Amid können erfindungsgemäss derart hergestellt werden, dass zunächst in der oben beschriebenen Weise das Lacton (IV) hergestellt und dieses mit einer wässrigen starken Base zur Hydroxysäure hydrolysiert oder mit Ammoniak in das Säureamid übergeführt wird.

Unter wässrigen starken Basen sind hier insbesondere Alkalilaugen zu verstehen, nicht dagegen wässrige Lösungen primärer oder sekundärer Amine.

Von den Verbindungen der ersten Gruppe (R² + R³ = =O) mit X = Chlor und R¹ = OH wird die Carbonsäure (R⁴ = ―OH) erfindungsgemäss hergestellt, indem in einer ersten Stufe Nicotin in der oben beschriebenen Weise durch mikrobiologische Transformation in das 5-Succinoyl-2-pyridon übergeführt und in einer zweiten Stufe mit Chlor in alkalischer Lösung chloriert wird. Die Chlorierung wird vorzugsweise derart durchgeführt, dass das 5-Succinoyl-2-pyridon mit einer starken Base, beispielsweise mit Natriumhydroxid, in das entsprechende Salz übergeführt und die Lösung auf einen pH von über 10 gebracht wird. Dann wird das Chlor eingeleitet und der pH durch Zudosieren weiterer Base über 10 gehalten. Die Reaktionstemperatur liegt vorzugsweise unterhalb der Raumtemperatur, beispielsweise bei -5 bis +10 °C. Anstelle der Einleitung von Chlorgas ist es auch möglich, die Chlorierung mit einer handelsüblichen Hypochloritlösung durchzuführen. Die Aufarbeitung kann aufübliche Weise erfolgen, wobei überschüssiges Chlor bzw. Hypochlorit und/oder eventuell gebildete N-Chlorverbindungen vorteilhaft durch Zugabe eines Reduktionsmittels wie beispielsweise Natriumsulfit zersetzt werden.

Die entsprechenden Ester (X= Cl, R¹ = OH, R² + R³ = =O und R⁴ = ―OR⁵, worin R⁵ C₁-C₄-Alkyl oder Benzyl ist) werden erfindungsgemäss auf dem gleichen Weg hergestellt, wobei zusätzlich noch eine Veresterung mit einem C₁―C₄-Alkanol oder Benzylalkohol als dritte Stufe angeschlossen wird.
Die Veresterung kann auf übliche Weise beispielsweise mit dem entsprechenden C₁―C₄-Alkohol oder Benzylalkohol und Schwefelsäure als Katalysator erfolgen.

Die Verbindungen der ersten Gruppe mit X = R¹ = Cl und R⁵ = C₁―C₄-Alkyl oder Benzyl werden erfindungsgemäss hergestellt, indem an den wie vorstehend beschrieben erhaltenen Estern mit R¹ = OH in einer weiteren Stufe die Hydroxygruppe gegen Chlor ausgetauscht wird. Dieser Austausch wird zweckmässig mit einem anorganischen Säurechlorid durchgeführt, vorzugsweise mit Phosphoroxychlorid.

Die entsprechende Carbonsäure, also die 4-(5,6-Dichlorpyridin-3-yl)-4-oxobuttersäure, kann aus den Estern durch Hydrolyse erhalten werden.

Die nachfolgenden Beispiele verdeutlichen die Herstellung der erfindungsgemässen Verbindungen sowie die Durchführung der erfindungsgemässen Verfahren.

### Beispiel 1

### Isolierung von Succinoylpyridon-bildenden Mikroorganismen

Aerobe (*S*)-Nicotin verwertende Mikroorganismen wurden im Minimal-Medium (Tabelle 1) mit (*S*)-Nicotin als einziger Kohlenstoff-Quelle angereichert. Die allgemeinen Techniken zur Isolation von Mikroorganismen sind beispielsweise in G. Drews, "Mikrobiologisches Praktikum", 4. Auflage, Springer Verlag, 1983, Seiten 1-84, beschrieben.
Als Inokulum wurden Proben aus der Abwasserreinigungsanlage Visp (Kanton Wallis, Schweiz), und einem Tabakfeld in Massongex (Kanton Wallis, Schweiz), verwendet. Die Anreicherungen wurden in Schüttelkolben bei 30 °C angezogen. Nach dreimaligem Ueberimpfen in frisches Medium wurden die Anreicherungen auf dem gleichen Medium mit Zusatz von 16 g Agar pro Liter ausgestrichen und bei 30 °C inkubiert. Nach mehrmaligem Ausstreichen auf Agarmedium konnten folgende Reinkulturen isoliert werden:
*Pseudomonas putida* DSM 8231 (hinterlegt am 26.04.1993 bei der DSM)
*Pseudomonas fluorescens* DSM 8235 (hinterlegt am 26.04.1993 bei der DSM)
*Pseudomonas putida* DSM 8236 (hinterlegt am 26.04.1993 bei der DSM)
*Variovorax paradoxus* (= *Alcaligenes paradoxus*) DSM 8244 (hinterlegt am 26.04.1993 bei der DSM)
*Pseudomonas sp.* DSM 8653 (hinterlegt am 27 10. 1993 bei der DSM)

**Tabelle 1**

| **Minimal-Medium** | |
|---|---|
| Zusammensetzung: | Konzentration [mg/l]: |
| (*S*)-Nicotin | 2000 |
| (NH₄)₂SO₄ | 2000 |
| Na₂HPO₄ | 2000 |
| KH₂PO₄ | 1000 |
| NaCl | 3000 |
| MgCl₂·6H₂O | 400 |
| CaCl₂·2H₂O | 14,5 |
| FeCl₃·6H₂O | 0,8 |
| ZnSO₄·7H₂O | 100·10⁻³ |
| MnCl₂·4H₂O | 90·10⁻³ |
| H₃BO₃ | 300·10⁻³ |
| CoCl₂·6H₂O | 200·10⁻³ |
| CuCl₂·2H₂O | 10·10⁻³ |
| NiCl₂·6H₂O | 20·10⁻³ |
| NaMoO₄·2H₂O | 30·10⁻³ |
| EDTANa₂·2H₂O | 5 |
| FeSO₄·7H₂O | 2 |

Der pH der Lösung wurde mit H₃PO₄ auf 7,0 eingestellt.

Um den Abbauweg von (*S*)-Nicotin in den Mikroorganismen zu bestimmen, wurden während der Wachstumsphase Proben aus einer Flüssigkultur entnommen. 2 Mikroliter der zellfreien Proben wurden auf Dünnschichtplatten (Merck, Kieselgel 60, F₂₅₄) aufgetragen und im folgenden Laufmittel entwickelt: Ethanol 55 ml; Chloroform 30 ml ; 25% wässriges Ammoniak 10 ml und Wasser 5 ml. Folgende R_{f}-Werte wurden bestimmt: (*S*)-Nicotin 0,92; 6-Hydroxy-(*S*)-nicotin 0,70; 4-(Pyridin-3-yl)-4-oxo-buttersäure 0,38; 5-Succinoyl-2-pyridon 0,31. Alle obengenannten Pseudomonaden bildeten Succinoylpyridon über 4-(Pyridin-3-yl)-4-oxo-buttersäure. *Variovorax paradoxus* bildete Succinoylpyridon über 6-Hydroxy-(*S*)-nicotin.

### Beispiel 2

### Biotransformation von (S)-Nicotin zu 5-Succinoyl-2-pyridon mit Pseudomonas sp. DSM 8653

400 ml Inokulum von *Pseudomonas sp.* DSM 8653 in Voll-Medium (Tabelle 2) wurden verwendet, um einen Fermenter mit 5 Liter Arbeitsvolumen anzuimpfen. Für die Fermentation wurde ebenfalls das Voll-Medium verwendet. Die Temperatur betrug 30 °C. Der pH wurde durch Zufuhr von 8,5% (*v/v*) H₃PO₄ und 1 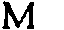 NaOH auf einen Wert von 7,0 geregelt. Die Luftzufuhr betrug zum Beginn der Fermentation 1 l/min und wurde nach 5 h auf 3 l/min erhöht. Nach 7 h betrug die optische Dichte der Suspension bei 650 um (OD₆₅₀) 4,0. Zu diesem Zeitpunkt wurde 50 g (*S*)-Nicotin zum Fermenter gegeben und gleichzeitig eine Pumpe eingeschaltet, die über eine Zeit von 15 h 1 Liter Feedlösung (Tabelle 3) kontinuierlich zum Fermenter zuführte. Die Nicotinkonzentration und die Konzentration der Nicotinmetabolite wurde sowohl mit Dünnschichtchromatographie (siehe Beispiel 1) als auch spektrophotometrisch bestimmt. Für Succinoylpyridon wurde bei 305 um in 0,1 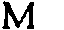 NaOH ein ε-Wert von 17000 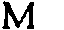 ⁻¹ cm⁻¹ gemessen. Bei der Beendigung der Fermentation nach 15 h betrug die OD₆₅₀ 9,2.
Zur Isolation des gebildeten Succinoylpyridons wurde der zellfreie Kulturüberstand mit Schwefelsäure bis zu einem pH von 2,5 angesäuert. Das ausgefallene Succinoylpyridon wurde durch Filtration isoliert und anschliessend getrocknet.

Insgesamt wurden 70 g (*S*)-Nicotin für die Biotransformation eingesetzt. Als Produkt wurden 77 g Succinoylpyridon isoliert, was einer Ausbeute von 91% entspricht.

**Tabelle 2**

| **Voll-Medium** | |
|---|---|
| Zusammensetzung | Konzentration [mg/l] |
| (*S*)-Nicotin | 2000 |
| Citronensäure | 800 |
| Hefeextrakt | 2000 |
| (NH₄)₂SO₄ | 2000 |
| Na₂HPO₄ | 2000 |
| KH₂PO₄ | 1000 |
| NaCl | 3000 |
| MgCl₂·6H₂O | 400 |
| CaCl₂·2H₂O | 14,5 |
| FeCl₃·6H₂O | 0,8 |
| ZnSO₄·7H₂O | 100·10⁻³ |
| MnCl₂·4H₂O | 90·10⁻³ |
| H₃BO₃ | 300·10⁻³ |
| CoCl₂·6H₂O | 200·10⁻³ |
| CuCl₂·2H₂O | 10·10⁻³ |
| NiCl₂·6H₂O | 20·10⁻³ |
| NaMoO₄·2H₂O | 30·10⁻³ |
| EDTANa₂·2H₂O | 5 |
| FeSO₄·7H₂O | 2 |

Der pH der Lösung wurde mit H₃PO₄ auf einen Wert von 7,0 gestellt.

**Tabelle 3**

| **Feed-Lösung** | |
|---|---|
| Zusammensetzung: | Konzentration [mg/l] |
| (*S*)-Nicotin | 20000 |
| Citronensäure | 8000 |
| Hefeextrakt | 20000 |

### Beispiele 3-5

### Biotransformation von (S)-Nicotin zu 5-Succinoyl-2-pyridon

Die Beispiele 3 bis 5 wurden entsprechend zu Beispiel 2 durchgeführt. Die Resultate sind in Tabelle 4 dargestellt:

**Tabelle 4**

| **Bsp.** | Stamm | Isolierte Menge Succinoylpyridon | Ausbeute |
|---|---|---|---|
| **3** | *P.putida* DSM 8231 | 45 g | 53% |
| **4** | *P. fluorescens* DSM 8235 | 40 g | 47% |
| **5** | *V. paradoxus* DSM 8244 | 62 g | 73% |

### Beispiel 6

### 4-(5-Chlor-1,6-dihydro-6-oxopyridin-3-yl)-4-oxobuttersäure

In 250 ml Wasser wurden bei Raumtemperatur 25 g (128 mmol) 5-Succinoyl-2-pyridon suspendiert. Durch Zugabe von 30%iger Natronlauge wurde ein pH von 10,5 eingestellt, wobei sich eine klare Lösung bildete. Die Lösung wurde auf 2 °C gekühlt. Chlorgas wurde langsam eingeleitet, wobei der pH durch Zudosieren von Natronlauge zwischen 10,2 und 10,5 und die Temperatur durch Kühlung unter 6 °C gehalten wurde. Insgesamt wurden 13,2 g Chlor und 72,4 g 30%ige Natronlauge verbraucht.
Zur Zerstörung von überschüssigem Chlor oder N-chlorierten Verbindungen wurden 19,2 g (152 mmol) Natriumsulfit zugesetzt und das Gemisch auf 40 °C erwärmt. Anschliessend wurde mit konz. Salzsäure bis zu einem pH von 2 angesäuert, wobei das gewünschte Produkt ausfiel. Die Suspension wurde noch 2 h bei Raumtemperatur gerührt, auf 2 °C abgekühlt und filtriert.
Ausbeute: 19,66 g (67%) weisser Festkörper.
   - ¹H-NMR (DMSO-d₆): δ: 2,52 (t, *J* = 6,8 Hz, 2H);
   3,08 (t, *J* =6,8 Hz, 2H);
   8,07 (d, *J* = 3,5 Hz, 1H);
   8,30 (d, *J* = 3,5 Hz, 1H);
   12,18 (br. s, 1H);
   12,77 (br. s, 1H).

### Beispiel 7

### 4-(5-Chlor-1,6-dihydro-6-oxopyridin-3-yl)-4-oxobuttersäuremethylester

In 730 ml Methanol wurden 43 g 4-(5-Chlor-1,6-dihydro-6-oxopyridin-3-yl)-4-oxobuttersäure (Rohprodukt, hergestellt nach Beispiel 6) suspendiert und mit 16 ml konz. Schwefelsäure versetzt. Das Gemisch wurde so erhitzt, dass innerhalb von 4 h ca. 100 ml Methanol abdestillierten. Das verbliebene Reaktionsgemisch wurde dann unter vermindertem Druck auf das halbe Volumen eingeengt und die so erhaltene Suspension auf 2 °C abgekühlt. Das Produkt wurde abfiltriert, mit 20 ml kaltem Methanol gewaschen und getrocknet.
Ausbeute: 38,1 g (83%) weisse Kristalle
Schmp.: 211,5-213,5 °C
   - ¹H-NMR (DMSO-d₆): δ: 2,60 (t, *J* = 7,8 Hz, 2H);
   3,15 (t, *J* = 7,8 Hz, 2H);
   3,59 (s, 3H);
   8,07 (d, *J* = 3,5 Hz, 1H);
   8,32 (d, *J* = 3,5 Hz, 1H);
   12,78 (br. s, 1H).

### Beispiel 8

### 4-(5-Chlor-1,6-dihydro-6-oxopyridin-3-yl)-4-oxobuttersäuremethylester

Diese Verbindung wurde auch in 28%iger Ausbeute durch Chlorierung von 5-Succinoyl-2-pyridon-methylester (erhalten analog zu Beispiel 7 aus 5-Succinoyl-2-pyridon und Methanol/Schwefelsäure) mit Chlor in Methanol erhalten. Als Hauptprodukt (70% Ausbeute) wurde hierbei der 4-(3,5-Dichlor-2-methoxy-6-oxo 1,2,3,6-tetrahydropyridin-3-yl)-4-oxobuttersäuremethylester mit den folgenden Daten erhalten:
¹H-NMR (CDCl₃): δ 2,69 (t, *J* = 6,9 Hz, 2H);
2,83 (m, 1H);
3,28 (m, 1H);
3,42 (s, 3H);
3,72 (s, 3H);
5,01 (dd, *J* =4,6 Hz, 1,8 Hz, 1H);
7,22 (d, *J* = 1,8 Hz, 1H);
8,57 (d, *J* = 4,6 Hz, 1H).
¹³C-NMR (CDCl₃): δ 28,02,
31,05
52,02,
55,86,
64,75,
86,59,
130,29,
133,50
160,00,
172,55,
196,27.

| | | | |
|---|---|---|---|
| Ber. | C 42,6 | H 4,2 | N 4,5 |
| Gef. | C 42,9 | H 4,3 | N 4,5 |

### Beispiel 9

### 4-(5,6-Dichlorpyridin-3-yl)-4-oxobuttersäuremethylester

Eine Suspension von 37 g (152 mmol) 4-(5-Chlor-1,6-dihydro-6-oxopyridin-3-yl)-4-oxobuttersäuremethylester in 230 ml Phosphoroxychlorid wurde 2,5 h auf 75 °C erwärmt. Anschliessend wurde das Phosphoroxychlorid im Vakuum abdestilliert. Der rötliche Rückstand wurde in 200 ml Dichlormethan aufgenommen, 100 ml Wasser zugegeben und der pH der wässrigen Phase mit 30%iger Natronlauge auf 8,5 eingestellt. Nach Phasentrennung wurde die wässrige Phase mit 100 ml Dichlormethan extrahiert. Die vereinigten organischen Phasen wurden mit Aktivkohle behandelt und eingedampft. Der Rückstand wurde aus 300 ml Diisopropylether unkristalllisiert und bei 2 °C filtriert.
Ausbeute: 32,83 g blassrosa Kristalle.
   Aus der Mutterlauge wurden weitere 1,76 g Produkt kristallin erhalten.
Gesamtausbeute: 87%
Schmp.: 85,2-86 °C
   - ¹H-NMR (CDCl₃): δ: 2,83 (t, *J* = 7,8 Hz, 2H);
   3,29 (t, *J* = 7,8 Hz, 2H);
   3,73 (s, 3H);
   8,33 (d, *J* = 2,4 Hz, 1H);
   8,87 (d, *J* =2,4 Hz, 1H).

### Beispiel 10

### 5-Succinoyl-2-pyridon-methylester (4-(1,6-Dihydro-6-oxopyridin-3-yl)-4-oxobuttersäuremethylester)

Eine Suspension von 27 g (138 mmol) 5-Succinoyl-2-pyridon in 400 ml Methanol wurde mit 10,4 g konzentrierter Schwefelsäure versetzt und 2 h auf 63 °C erhitzt, wobei 100 ml Methanol abdestillierten. Die entstandene braune Lösung wurde nach dem Abkühlen auf Raumtemperatur in den Kühlschrank gestellt, wo das Produkt auskristallisierte. Der Ester wurde abfiltriert, mit kaltem Methanol gewaschen und im Vakuum getrocknet.
Ausbeute: 23,5 g (81%) weisse Kristalle.
Schmp.: 167-168 °C
   - ¹H-NMR (DMSO-d₆): δ: 2,59 (t, *J* = 7,5 Hz, 2H);
   3,13 (t, *J* = 7,5 Hz, 2H);
   3,58 (s, 3H);
   6,37 (d, *J* = 12 Hz, 1H);
   7,86 (dd, *J* = 12 Hz, 3,5 Hz, 1H);
   8,27(d, *J* = 3,5 Hz, 1H);
   12,14 (br. s, 1H).
MS (EI) *m/z* (%): 209 [M⁺], 178, 150, 122 (100 %)

### Beispiel 11

### 5-Succinoyl-2-pyridon-ethylester (4-(1,6-Dihydro-6-oxopyridin-3-yl)-4-oxobuttersäureethylester)

Der Ester wurde analog zum Methylester (Beispiel 10) mit Ethanol/Schwefelsäure hergestellt.
Schmp.: 121,8-122,7°C
   - ¹H-NMR (DMSO-d₆): δ: 1,18 (t, *J* = 8,2 Hz, 3H);
   2,58 (t, *J* = 7,5 Hz, 2H);
   3,11 (t, *J* = 7,5 Hz, 2H);
   4,05 (q, *J* = 8,2 Hz, 2H);
   6,37 (d, *J* = 12 Hz, 1H);
   7,86 (dd, *J* = 12 Hz, 3,5 Hz, 1H);
   8,26 (d, *J* = 3,5 Hz, 1H);
   12,18 (br.s, 1H).

### Beispiel 12

### 2-Chlor-5-succinoylpyridin-methylester (4-(6-Chlorpyridin-3-yl)-4-oxobuttersäuremethylester)

In 110 ml Phosphoroxychlorid wurden 25 g (119 mmol) 5-Succinoyl-2-pyridon-methylester (hergestellt nach Beispiel 10) bei 20 °C suspendiert. Das Gemisch wurde vorsichtig auf 70 °C erwärmt und 1 h auf dieser Temperatur gehalten. Das Phosphoroxychlorid wurde unter vermindertem Druck abdestilliert. Der braune, ölige Rückstand wurde in je 100 ml Toluol und Wasser aufgenommen, die wässrige Phase mit 30%iger Natronlauge auf pH 9 eingestellt und zweimal mit Toluol extrahiert.
Die vereinigten Toluolextrakte wurden eingedampft. Der Rückstand, ein rötliches Öl (26,15 g), wurde nach Behandlung mit Aktivkohle aus Diisopropylether kristallisiert.
Ausbeute: 24,14 g (89%) weisse Kristalle
Schmp.: 73,4-74 °C
   - ¹H-NMR (CDCl₃): δ: 2,81 (t, *J* = 7,5 Hz, 2H);
   3,32 (t, *J* = 7,5 Hz, 2H);
   3,72 (s, 3H);
   7,47 (d, *J* = 10,2 Hz, 1H)
   8,23 (dd, *J* = 10,2 Hz, 3,6 Hz, 1H);
   8,97 (d, *J* = 3,6 Hz, 1H).

### Beispiel 13

### 6-(1,6-Dihydro-6-oxopyridin-3-yl)-2,3,4,5-tetrahydropyridazin-3-on

In 67 ml Ethanol wurden 5 g (23,9 mmol) 5-Succinoyl-2-pyridon-methylester (hergestellt nach Beispiel 10) suspendiert und mit 2,4 g (48 mmol) Hydrazinhydrat versetzt. Das Gemisch wurde auf 70 °C erwärmt, wobei das Edukt in Lösung ging. Die farblose Lösung wurde über Nacht am Rückfluss gekocht, wobei sich ein dicker weisser Niederschlag bildete. Dieser wurde nach dem Abkühlen abfiltriert, mit Ethanol gewaschen und im Vakuum getrocknet.
Ausbeute: 4,33 g (95%)
Schmp.: >260 °C
¹H-NMR (DMSO-d₆): δ 2,38 (t, *J* = 9,6 Hz, 2H);
2,80 (t, *J* = 9,6 Hz, 2H);
6,38 (d, *J* = 12 Hz, 1H);
7,69 (d, *J* = 3 Hz, 1H);
7,90 (dd, *J* = 12 Hz, 3 Hz, 1H);
10,80 (s, 1H),
11,84 (br.s, 1H).

| | | | |
|---|---|---|---|
| Ber. | C 56,54 | H 4,75 | N 21,98 |
| Gef. | C 56,44 | H 4,75 | N 21,61 |

### Beispiel 14

### 6-(6-Chlorpyridin-3-yl)-2,3,4,5-tetrahydropyridazin-3-on

Zu einer Lösung von 3 g (13,2 mmol) 2-Chlor-5-succinoylpyridin-methylester (hergestellt nach Beispiel 12) in 55 ml eines Gemischs aus Toluol und Methanol (7 : 4) wurden bei Raumtemperatur 1,13 g (22,6 mmol) Hydrazinhydrat gegeben. Die Mischung wurde 5 h gerührt, wobei das Produkt allmählich ausfiel. Die Suspension wurde auf 2 °C gekühlt und filtriert. Der Niederschlag wurde mit wenig kaltem Toluol/Methanol-Gemisch gewaschen und getrocknet.
Ausbeute: 1,86 g (67%) weisse Nadeln
   Durch Eindampfen der Mutterlauge wurde 1 g eines Rückstands erhalten, der im wesentlichen ebenfalls aus dem gewünschten Produkt bestand.
Schmp.: 210,8-211,8 °C
   - ¹H-NMR (DMSO-d₆): δ: 2,50 (t, *J* = 9,6 Hz, 2H);
   2,98 (t, *J* = 9,6 Hz, 2H);
   7,58 (d, *J* = 10,2 Hz, 1H);
   8,17 (dd, *J* = 10,2 Hz, 3,6 Hz, 1H);
   8,74 (d, *J* = 3,6 Hz, 1H);
   11,11 (s, 1H).

### Beispiel 15

### 5-(6-Chlorpyridin-3-yl)-tetrahydrofuran-2-on

Zu einer Lösung von 15 g (66 mmol) 4-(6-Chlorpyridin-3-yl)-4-oxobuttersäuremethylester (hergestellt nach Beispiel 12) in 220 ml Methanol wurden bei 25 °C innerhalb von 20 min portionsweise 1,97 g (52 mmol) Natriumborhydrid gegeben. Das Reaktionsgemisch wurde 1 h gerührt, mit 200 ml Toluol versetzt und das Methanol abdestilliert. Dann wurde das Gemisch noch 1 h auf 100 °C erhitzt, um die Lactonbildung zu vervollständigen. Anschliessend wurde das Gemisch filtriert und das Filtrat eingedampft. Der Rückstand (13,3 g bräunliches Öl) wurde aus 70 ml Diisopropylether kristallisiert.
Ausbeute: 11,95 g (92%) weisser Festkörper
Schmp.: 52,5-53°C
   - ¹H-NMR (CDCl₃): δ: 2,20 (m, 1H);
   2,72 (m, 2H);
   2,76 (m, 1H);
   5,54 (m, 1H);
   7,49 (d, *J* = 10,2 Hz, 1H);
   7,68 (dd, *J* = 10,2 Hz, 3 Hz, 1H);
   8,38 (d, *J* = 3 Hz, 2H).
   - ¹³C-NMR (CDCl₃): δ: 28,77,
   30,63,
   78,19,
   124,55,
   134,12,
   136,10,
   147,13,
   151,69,
   176,02.

### Beispiel 16

### 4-(6-Chlorpyridin-3-yl)-4-hydroxybutyramid

In 30 ml 25%iger wässriger Ammoniaklösung wurden 3,6 g 5-(6-Chlorpyridin-3-yl)tetrahydrofuran-2-on (hergestellt nach Beispiel 15) gelöst und 1,5 h auf 45 °C erwärmt. Die Lösung wurde im Vakuum eingedampft. Der ölige Rückstand (4,65 g) wurde im Vakuum über Phosphorpentoxid getrocknet.
Ausbeute: 3,9 g (ca. 100%) gummiartiges Produkt
   - ¹H-NMR (DMSO-d₆): δ: 1,85 (m, 2H);
   2,12 (t, *J* = 7,5 Hz, 2H);
   4,63 (m, 1H);
   5,57 (d, *J* = 6 Hz, 1H);
   6,76 (br. s, 1H);
   7,30 (br. s, 1H);
   7,4g (d, *J* = 9,6 Hz, 1H);
   7,80 (dd, *J* = 9,6 Hz, 3,5 Hz, 1H);
   8,35 (d, *J* = 3,5 Hz, 1H).

### Beispiel 17

### 4-(6-Chlorpyridin-3-yl)-4-hydroxybuttersäure

Die Säure wurde in praktisch quantitativer Ausbeute durch Umsetzung von 5-(6-Chlorpyridin-3-yl)tetrahydrofuran-2-on (hergestellt nach Beispiel 15) mit wässrigem Natriumhydroxid bei pH 13 und Raumtemperatur erhalten. Nach Neutralisation mit Salzsäure wurde das Reaktionsgemisch eingedampft und der Rückstand aus Ethanol umkristallisiert. Die Säure fiel so in Form weisser Kristalle an.
- ¹H-NMR (CDCl₃): δ: 2,06 (m, 2H);
2,54 (m, 2H);
4,85 (t, 1H),
7,33 (d, 1H);
7,73 (dd, 1H);
8,38 (d, 1H).
- ¹³C-NMR (DMSO-d₆): δ: 34,97,
35,35,
70,38,
123,5,
137,23,
141,63,
147,60,
148,14,
178,19.
- MS der Bis-trimethylsilyl-Verbindung: *m/z*:: 359/361 (M⁺), 214/216 (100%)
Die Intensitäten entsprechen dem Isotopenverhältnis von Chlor.

## Patentansprüche

1. Substituierte Pyridine der allgemeinen Formel 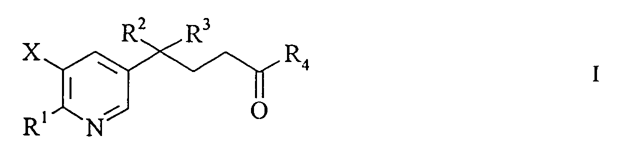 worin R¹ Hydroxy oder Chlor und
a) X Wasserstoff oder Chlor, R² und R³ zusammen =O, R⁴ eine Gruppe der Formel -OR⁵ und R⁵ Wasserstoff, C₁-C₄-Alkyl oder Benzyl oder
b) X Wasserstoff und R², R³ und R⁴ zusammen =N―NH― oder
c) X und R² Wasserstoff und R³ und R⁴ zusammen ―O― oder
d) X und R² Wasserstoff, R³ Hydroxy und R⁴ Amino oder Hydroxy bedeuten,
ausgenommen die Verbindung mit X = H, R¹ = R⁴ = ―OH, R² + R³ = =O.

2. Verfahren zur Herstellung 2,5-disubstituierter Pyridine gemäss Anspruch 1, worin X Wasserstoff ist und R², R³ und R⁴ zusammen eine =N―NH― -Gruppe bilden, dadurch gekennzeichnet, dass in einer ersten Stufe Nicotin mit einem Mikroorganismus der Gattung *Pseudomonas* oder *Variovorax* zu 5-Succinoyl-2-pyridon oxidiert, dieses mit einem C₁―C₄-Alkanol oder Benzylalkohol zu dem entsprechenden C₁―C₄-Alkyl- oder Benzylester der Formel 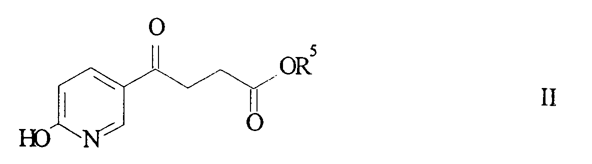 worin R⁵ C₁―C₄-Alkyl oder Benzyl ist, verestert, gegebenenfalls durch Austausch der Hydroxygruppe gegen Chlor in die entsprechende Chlorverbindung übergeführt und schliesslich mit Hydrazin zum Pyridazinonderivat der Formel 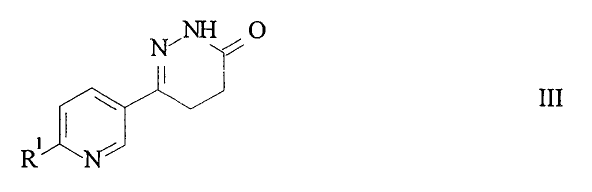 cyclisiert wird.

3. Verfahren nach Anspruch 2, dadurch gekennzeichnet, dass für die mikrobiologische Oxidation ein Mikroorganismus aus der Gruppe *Pseudomonas putida* DSM 8231, *Pseudomonas putida* DSM 8236, *Pseudomonas fluorescens* DSM *8235, Pseudomonas sp.* DSM 8653, *Variovorax paradoxus* (= *Alcaligenes paradoxus*) DSM 8244 eingesetzt wird.

4. Verfahren nach Anspruch 2 oder 3, dadurch gekennzeichnet, dass der C₁―C₄-Alkyl- oder Benzylester (II) durch Austausch der Hydroxygruppe mit Phosphoroxychlorid in die Chlorverbindung übergeführt wird.

5. Verfahren zur Herstellung 2,5-disubstituierter Pyridine gemäss Anspruch 1, worin X Wasserstoff, R¹ Chlor, R² Wasserstoffund R³ und R⁴ zusammen ―O― sind, dadurch gekennzeichnet, dass in einer ersten Stufe Nicotin mit einem Mikroorganismus der Gattung *Pseudomonas* oder *Variovorax* zu 5-Succinoyl-2-pyridon oxidiert, dieses mit einem C₁―C₄-Alkanol oder Benzylalkohol zu dem entsprechenden C₁―C₄-Alkyl- oder Benzylester der Formel 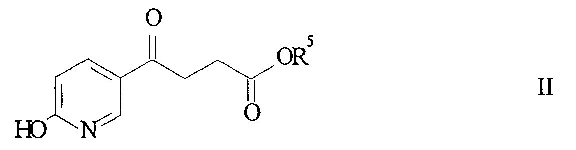 worin R⁵ C₁―C₄-Alkyl oder Benzyl ist, verestert, durch Austausch der Hydroxygruppe gegen Chlor in die entsprechende Chlorverbindung übergeführt, reduziert und zu dem Lacton der Formel 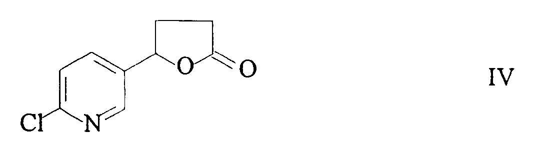 cyclisiert wird.

6. Verfahren nach Anspruch 5, dadurch gekennzeichnet, dass für die mikrobiologische Oxidation ein Mikroorganismus aus der Gruppe *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 eingesetzt wird.

7. Verfahren nach Anspruch 5 oder 6, dadurch gekennzeichnet, dass der C₁―C₄-Alkyl- oder Benzylester (II) durch Austausch der Hydroxygruppe mit Phosphoroxychlorid in die Chlorverbindung übergeführt wird.

8. Verfahren nach einem der Ansprüche 5 bis 7, dadurch gekennzeichnet, dass die Reduktion des Esters (II) zum Lacton (IV) mit Natriumborhydrid als Reduktionsmittel durchgeführt wird.

9. Verfahren zur Herstellung 2,5-disubstituierter Pyridine gemäss Anspruch 1, worin X Wasserstoff, R¹ Chlor, R² Wasserstoff, R³ Hydroxy und R⁴ Amino oder Hydroxy ist, dadurch gekennzeichnet, dass in einer ersten Stufe Nicotin mit einem Mikroorganismus der Gattung *Pseudomonas* oder *Variovorax* zu 5-Succinoyl-2-pyridon oxidiert, dieses mit einem C₁―C₄-Alkanol oder Benzylalkohol zu dem entsprechenden C₁―C₄-Alkyl- oder Benzylester der Formel 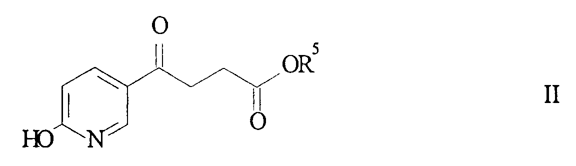 worin R⁵ C₁―C₄-Alkyl oder Benzyl ist, verestert, durch Austausch der Hydroxygruppe gegen Chlor in die entsprechende Chlorverbindung übergeführt, zu dem Lacton der Formel 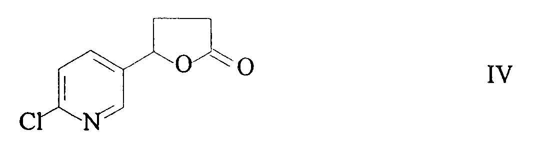 reduziert und cyclisiert und schliesslich mit Ammoniak oder einer wässrigen starken Base in das Amid beziehungsweise die Säure der Formel 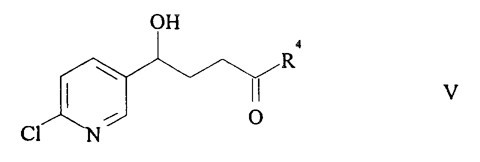 worin R⁴ die oben genannte Bedeutung hat, übergeführt wird.

10. Verfahren nach Anspruch 9, dadurch gekennzeichnet, dass für die mikrobiologische Oxidation ein Mikroorganismus aus der Gruppe *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 eingesetzt wird.

11. Verfahren nach Anspruch 9 oder 10, dadurch gekennzeichnet, dass der C₁―C₄-Alkyl- oder Benzylester (II) durch Austausch der Hydroxygruppe mit Phosphoroxychlorid in die Chlorverbindung übergeführt wird.

12. Verfahren nach einem der Ansprüche 9 bis 11, dadurch gekennzeichnet, dass die Reduktion des Esters (II) zum Lacton (IV) mit Natriumborhydrid als Reduktionsmittel durchgeführt wird.

13. Verfahren zur Herstellung eines 2,3,5-trisubstituierten Pyridins gemäss Anspruch 1, worin X Chlor, R¹ Hydroxy, R² und R³ zusammen =O und R⁴ Hydroxy bedeutet, dadurch gekennzeichnet, dass in einer ersten Stufe Nicotin mit einem Mikroorganismus der Gattung *Pseudomonas* oder *Variovorax* zu 5-Succinoyl-2-pyridon oxidiert und dieses in einer zweiten Stufe mit Chlor in alkalischer Lösung zu 3-Chlor-5-succinoyl-2-pyridon chloriert wird.

14. Verfahren zur Herstellung von 2,3,5-trisubstituierten Pyridinen gemäss Anspruch 1, worin X Chlor, R¹ Hydroxy, R² und R³ zusammen =O, R⁴ OR⁵ und R⁵ C₁―C₄-Alkyl oder Benzyl bedeutet, dadurch gekennzeichnet, dass in einer ersten Stufe Nicotin mit einem Mikroorganismus der Gattung *Pseudomonas* oder *Variovorax* zu 5-Succinoyl-2 pyridon oxidiert, dieses in einer zweiten Stufe mit Chlor in alkalischer Lösung zu 3 Chlor-5-succinoyl-2-pyridon (I, X = Cl, R¹ = OH, R² + R³ = =O, R⁴ = OH) chloriert und schliesslich in einer dritten Stufe mit einem C₁―C₄-Alkanol oder Benzylalkohol zum C₁―C₄-Alkyl- oder Benzylester verestert wird.

15. Verfahren zur Herstellung von 2,3,5-trisubstituierten Pyridinen gemäss Anspruch l, worin X Chlor, R¹ Chlor, R² und R³ zusammen =O, R⁴ OR⁵ und R⁵ C₁―C₄-Alkyl oder Benzyl bedeutet, dadurch gekennzeichnet, dass in einer ersten Stufe Nicotin mit einem Mikroorganismus der Gattung *Pseudomonas* oder *Variovorax* zu 5-Succinoyl-2-pyridon oxidiert, dieses in einer zweiten Stufe mit Chlor in alkalischer Lösung zu 3 Chlor-5-succinoyl-2-pyridon (I, X = Cl, R¹ = OH, R² + R³ = =O, R⁴ = OH) chloriert, in einer dritten Stufe mit einem C₁―C₄-Alkanol oder Benzylalkohol zum C₁―C₄-Alkyl- oder oder Benzylester verestert und schliesslich durch Austausch der Hydroxygruppe gegen Chlor in die 2,3-Dichlorverbindung übergeführt wird.

16. Verfahren nach Anspruch 15, dadurch gekennzeichnet, dass als Reagens zum Austausch der Hydroxygruppe gegen Chlor Phosphoroxychlorid eingesetzt wird.

17. Verfahren nach einem der Ansprüche 13 bis dadurch gekennzeichnet, dass für die Oxidation des Nicotins ein Mikroorganismus aus der Gruppe *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 eingesetzt wird.

## Claims

1. Substituted pyridines corresponding to the general formula 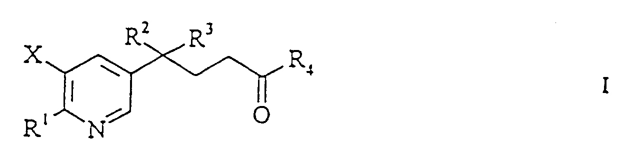 wherein R¹ denotes hydroxy or chlorine and
a) X denotes hydrogen or chlorine, R² and R³ together denote =O, R⁴ denotes a group corresponding to the formula -OR⁵ and R⁵ is hydrogen, C₁-C₄-alkyl or benzyl or
b) X denotes hydrogen and R², R³ and R⁴ together denote =N-NH- or
c) X and R² denote hydrogen and R³ and R⁴ together denote -O- or
d) X and R² denote hydrogen, R³ denotes hydroxy and R⁴ denotes amino or hydroxy,
excluding the compound wherein X is H, R¹ and R⁴ are identical and are -OH, R² + R³ are =O.

2. Process for the preparation of 2,5-disubstituted pyridines according to claim 1, wherein X is hydrogen and R², R³ and R⁴ together form an =N-NH- group, characterised in that in a first step nicotine, by means of a microorganism of the genus *Pseudomonas* or *Variovorax,* is oxidised to 5-succinoyl-2-pyridone, this is esterified with a C₁-C₄-alkanol or benzyl alcohol to the corresponding C₁-C₄-alkyl or benzyl ester having the formula 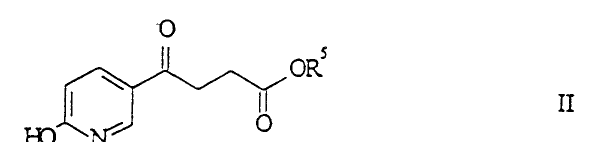 wherein R⁵ is C₁-C₄-alkyl or benzyl, and optionally converted to the corresponding chlorine compound by exchange of the hydroxyl group for chlorine and finally cyclised with hydrazine to the pyridazinone derivative having the formula

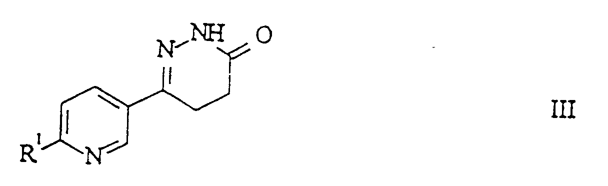

3. Process according to claim 2, characterised in that a microorganism from the group *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 is used for the microbiological oxidation.

4. Process according to claim 2 or 3, characterised in that the C₁-C₄-alkyl or benzyl ester (II) is converted to the chlorine compound by exchange of the hydroxyl group using phosphorus oxychloride.

5. Process for the preparation of 2,5-disubstituted pyridines according to claim 1, wherein X is hydrogen, R¹ is chlorine, R² is hydrogen and R³ and R⁴ together are -O-, characterised in that in a first step nicotine, by means of a microorganism of the genus *Pseudomonas* or *Variovorax,* is oxidised to 5-succinoyl-2-pyridone, this is esterified with a C₁-C₄-alkanol or benzyl alcohol to the corresponding C₁-C₄-alkyl or benzyl ester having the formula 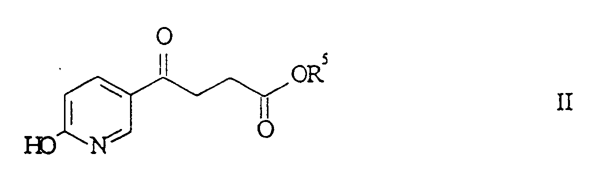 wherein R⁵ is C₁-C₄-alkyl or benzyl, and converted to the corresponding chlorine compound by exchange of the hydroxyl group for chlorine, reduced and cyclised to the lactone having the formula

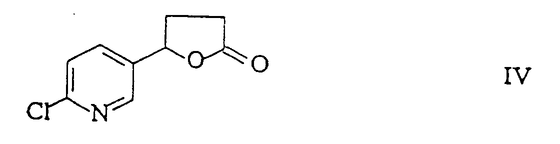

6. Process according to claim 5, characterised in that a microorganism from the group *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 is used for the microbiological oxidation.

7. Process according to claim 5 or 6, characterised in that the C₁-C₄-alkyl or benzyl ester (II) is converted to the chlorine compound by exchange of the hydroxyl group using phosphorus oxychloride.

8. Process according to one of claims 5 to 7, characterised in that the reduction of the ester (II) to the lactone (IV) is carried out using sodium borohydride as reducing agent.

9. Process for the preparation of 2,5-disubstituted pyridines according to claim 1, wherein X is hydrogen, R¹ is chlorine, R² is hydrogen, R³ is hydroxy and R⁴ is amino or hydroxy, characterised in that in a first step nicotine, by means of a microorganism of the genus *Pseudomonas* or *Variovorax,* is oxidised to 5-succinoyl-2-pyridone, this is esterified with a C₁-C₄-alkanol or benzyl alcohol to the corresponding C₁-C₄-alkyl or benzyl ester having the formula 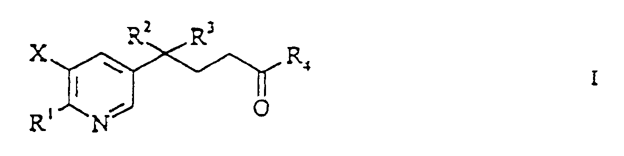 wherein R⁵ is C₁-C₄-alkyl or benzyl, and converted to the corresponding chlorine compound by exchange of the hydroxyl group for chlorine, reduced and cyclised to the lactone having the formula 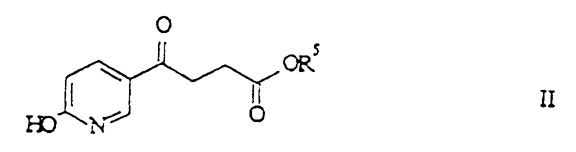 and finally, by means of ammonia or an aqueous strong base, is converted to the amide or to the acid having the formula 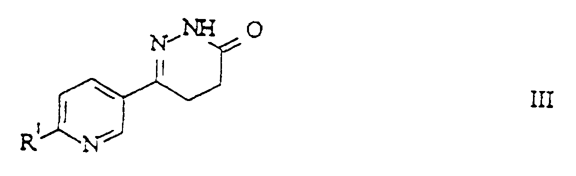 wherein R⁴ has the meaning given above.

10. Process according to claim 9, characterised in that a microorganism from the group *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 is used for the microbiological oxidation.

11. Process according to claim 9 or 10, characterised in that the C₁-C₄-alkyl or benzyl ester (II) is converted to the chlorine compound by exchange of the hydroxyl group using phosphorus oxychloride.

12. Process according to one of claims 9 to 11, characterised in that the reduction of the ester (II) to the lactone (IV) is carried out using sodium borohydride as reducing agent.

13. Process for the preparation of a 2,3,5-substituted pyridine according to claim 1, wherein X denotes chlorine, R¹ denotes hydroxy, R² and R³ together denote =O and R⁴ denotes hydroxy, characterised in that in a first step nicotine, by means of a microorganism of the genus *Pseudomonas* or *Variovorax,* is oxidised to 5-succinoyl-2-pyridone and in a second step this is chlorinated to 3-chloro-5-succinoyl-2-pyridone using chlorine in alkaline solution.

14. Process for the preparation of 2,3,5-trisubstituted pyridines according to claim 1, wherein X denotes chlorine, R¹ denotes hydroxy, R² and R³ together denote =O, R⁴ OR⁵ and R⁵ denote C₁-C₄-alkyl or benzyl, characterised in that in a first step nicotine, by means of a microorganism of the genus *Pseudomonas* or *Variovorax,* is oxidised to 5-succinoyl-2-pyridone, in a second step this is chlorinated to 3-chloro-5-succinoyl-2-pyridone (I, X is Cl, R¹ is OH, R² + R³ are =O, R⁴ is OH) using chlorine in alkaline solution and finally in a third step is esterified with a C₁-C₄-alkanol or benzyl alcohol to the C₁-C₄-alkyl or benzyl ester.

15. Process for the preparation of 2,3,5-trisubstituted pyridines according to claim 1, wherein X denotes chlorine, R¹ denotes chlorine, R² and R³ together denote =O, R⁴ OR⁵ and R⁵ denote C₁-C₄-alkyl or benzyl, characterised in that in a first step nicotine, by means of a microorganism of the genus *Pseudomonas* or *Variovorax,* is oxidised to 5-succinoyl-2-pyridone, in a second step this is chlorinated to 3-chloro-5-succinoyl-2-pyridone (I, X is Cl, R¹ is OH, R² + R³ are =O, R⁴ is OH) using chlorine in alkaline solution, in a third step is esterified with a C₁-C₄-alkanol or benzyl alcohol to the C₁-C₄-alkyl or benzyl ester and finally is converted to the 2,3-dichloro compound by exchange of the hydroxyl group for chlorine.

16. Process according to claim 15, characterised in that phosphorus oxychloride is used as reagent for the exchange of the hydroxyl group for chlorine.

17. Process according to one of claims 13 to 16, characterised in that a microorganism from the group *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244 is used for the oxidation of the nicotine.

## Revendications

1. Pyridines substituées de formule générale 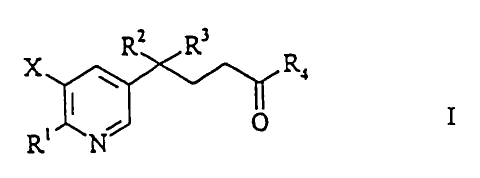 dans laquelle R¹ est un groupe hydroxy ou un atome de chlore et
a) X est un atome d'hydrogène ou de chlore, R² et R³ représentent ensemble =O, R⁴ est un groupe de formule -OR⁵ et R⁵ est un atome d'hydrogène ou un groupe alkyle en C₁-C₄ ou benzyle, ou
b) X est un atome d'hydrogène et R², R³ et R⁴ représentent ensemble =N-NH-, ou
c) X et R² sont des atomes d'hydrogène et R³ et R⁴ représentent ensemble -O-, ou
d) X et R² sont des atomes d'hydrogène, R³ est un groupe hydroxy et R⁴ est un groupe amino ou hydroxy,
à l'exception du composé dans lequel X = H, R¹ = R⁴= -OH, R² + R³= =O.

2. Procédé de préparation de pyridines disubstituées en 2,5 selon la revendication 1 , dans lesquelles X est un atome d'hydrogène et R², R³ et R⁴ forment ensemble un groupe =N-NH-, caractérisé en ce que, dans une première étape, on oxyde de la nicotine avec un micro-organisme du genre *Pseudomonas* ou *Variovorax* pour former la 5-succinoyl-2-pyridone, que l'on estérifie avec un alcanol en C₁-C₄ ou l'alcool benzylique pour obtenir l'ester d'alkyle en C₁-C₄ ou de benzyle correspondant de formule 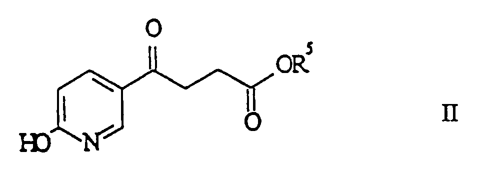 dans laquelle R⁵ est un groupe alkyle en C₁-C₄ ou benzyle, on transforme éventuellement ce composé en le composé chloré correspondant par échange du groupe hydroxy contre du chlore, et enfin on le cyclise avec de l'hydrazine pour obtenir le dérivé de pyridazinone de formule

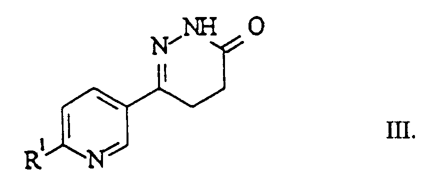

3. Procédé selon la revendication 2, caractérisé en ce que l'on utilise pour l'oxydation microbiologique un micro-organisme du groupe constitué par *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244.

4. Procédé selon la revendication 2 ou 3, caractérisé en ce que l'on transforme l'ester d'alkyle en C₁-C₄ ou de benzyle (II) en le composé chloré par échange du groupe hydroxy avec de l'oxychlorure de phosphore.

5. Procédé de préparation d'une pyridine disubstituée en 2,5 selon la revendication 1 dans laquelle X est un atome d'hydrogène, R¹ est un atome de chlore, R² est un atome d'hydrogène et R³ et R⁴ sont ensemble -O-, caractérisé en ce que, dans une première étape, on oxyde de la nicotine avec un micro-organisme du genre *Pseudomonas* ou *Variovorax* pour former la 5-succinoyl-2-pyridone, que l'on estérifie avec un alcanol en C₁-C₄ ou l'alcool benzylique pour obtenir l'ester d'alkyle en C₁-C₄ ou de benzyle correspondant de formule 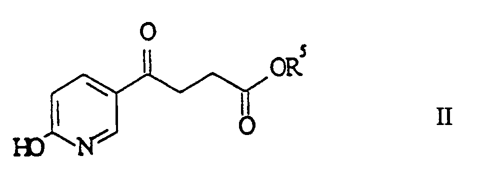 dans laquelle R⁵ est un groupe alkyle en C₁-C₄ ou benzyle, on transforme ce composé en le composé chloré correspondant par échange du groupe hydroxy contre du chlore, on le réduit et on le cyclise pour obtenir la lactone de formule

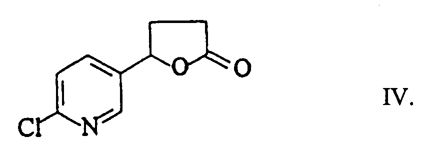

6. Procédé selon la revendication 5, caractérisé en ce que l'on utilise pour l'oxydation microbiologique un micro-organisme du groupe constitué par *Pseudomonas putida* DSM 8231 *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244.

7. Procédé selon la revendication 5 ou 6, caractérisé en ce que l'on transforme l'ester d'alkyle en C₁-C₄ ou de benzyle (II) en le composé chloré par échange du groupe hydroxy avec de l'oxychlorure de phosphore.

8. Procédé selon l'une des revendications 5 à 7, caractérisé en ce que la réduction de l'ester (II) en la lactone (IV) s'effectue avec du borohydrure de sodium comme agent réducteur.

9. Procédé de préparation de pyridines disubstituées en 2,5 selon la revendication 1, dans lesquelles X est un atome d'hydrogène, R¹ est un atome de chlore, R² est un atome d'hydrogène, R³ est un groupe hydroxy et R⁴ est un groupe amino ou hydroxy, caractérisé en ce que, dans une première étape, on oxyde de la nicotine avec un micro-organisme du genre *Pseudomonas* ou *Variovorax* pour former la 5-succinoyl-2-pyridone, que l'on estérifie avec un alcanol en C₁-C₄ ou l'alcool benzylique pour obtenir l'ester d'alkyle en C₁-C₄ ou de benzyle correspondant de formule 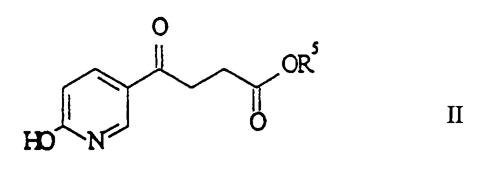 dans laquelle R⁵ est un groupe alkyle en C₁-C₄ ou benzyle, on transforme ce composé en le composé chloré correspondant par échange du groupe hydroxy contre du chlore, on le réduit et on le cyclise pour obtenir la lactone de formule 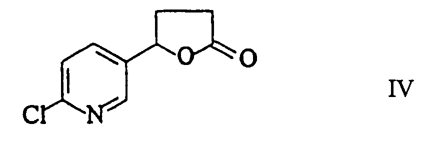 que l'on transforme finalement avec de l'ammoniac ou une base forte aqueuse en l'amide ou en l'acide respectifs de formule 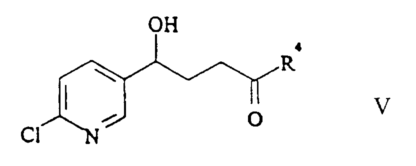 dans laquelle R⁴ a la signification indiquée ci-dessus.

10. Procédé selon la revendication 9, caractérisé en ce que l'on utilise pour l'oxydation microbiologique un micro-organisme du groupe constitué par *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244.

11. Procédé selon la revendication 9 ou 10, caractérisé en ce que l'on transforme l'ester d'alkyle en C₁-C₄ ou de benzyle (II) en le composé chloré par échange du groupe hydroxy avec de l'oxychlorure de phosphore.

12. Procédé selon l'une des revendications 9 à 11, caractérisé en ce que la réduction de l'ester (II) en la lactone (IV) s'effectue avec du borohydrure de sodium comme agent réducteur.

13. Procédé de préparation d'une pyridine trisubstituée en 2,3,5 selon la revendication 1, dans laquelle X est un atome de chlore, R¹ est un groupe hydroxy, R² et R³ sont ensemble =O et R⁴ est un groupe hydroxy, caractérisé en ce dans une première étape, on oxyde de la nicotine avec un micro-organisme du genre *Pseudomonas* ou *Variovorax* pour former la 5-succinoyl-2-pyridone et on la chlore dans une seconde étape avec du chlore en solution basique pour obtenir la 3-chloro-5-succinoyl-2-pyridone.

14. Procédé de préparation de pyridines trisubstituées en 2,3,5 selon la revendication 1, dans lesquelles X est un atome de chlore, R¹ est un groupe hydroxy, R² et R³ sont ensemble =O, R⁴ est un groupe OR⁵ et R⁵ est un groupe alkyle en C₁-C₄ ou benzyle, caractérisé en ce que, dans une première étape, on oxyde de la nicotine avec un micro-organisme du genre *Pseudomonas* ou *Variovorax* pour former la 5-succinoyl-2-pyridone, que l'on chlore dans une deuxième étape avec du chlore en solution basique pour obtenir la 3-chloro-5-succinoyl-2-pyridone (I, X = Cl, R¹ = OH, R² + R³ = =O, R⁴ = OH), et finalement, dans une troisième étape, on l'estérifie avec un alcanol en C₁-C₄ ou de l'alcool benzylique pour obtenir l'ester d'alkyle en C₁-C₄ ou de benzyle.

15. Procédé de préparation de pyridines trisubstituées en 2,3,5 selon la revendication 1, dans lesquelles X est un atome de chlore, R¹ est un atome de chlore, R² et R³ sont ensemble =O, R⁴ est un groupe OR⁵ et R⁵ est un groupe alkyle en C₁-C₄ ou benzyle, caractérisé en ce que, dans une première étape, on oxyde de la nicotine avec un micro-organisme du genre *Pseudomonas* ou *Variovorax* pour former la 5-succinoyl-2-pyridone, que l'on chlore dans une deuxième étape avec du chlore en solution basique pour obtenir la 3-chloro-5-succinoyl-2-pyridone (I, X = Cl, R¹ = OH, R² + R³ = =O, R⁴ = OH), on l'estérifie dans une troisième étape avec un alcanol en C₁-C₄ ou de l'alcool benzylique pour obtenir l'ester d'alkyle en C₁-C₄ ou de benzyle et on transforme finalement ce dernier en le composé 2,3-dichloro par échange du groupe hydroxy contre du chlore.

16. Procédé selon la revendication 15, caractérisé en ce que l'on utilise de l'oxychlorure de phosphore comme réactif pour l'échange du groupe hydroxy contre du chlore.

17. Procédé selon l'une des revendications 13 à 16, caractérisé en ce que, pour l'oxydation de la nicotine, on utilise un micro-organisme du groupe constitué par *Pseudomonas putida* DSM 8231, *Pseudomonas putida* DSM 8236, *Pseudomonas fluorescens* DSM 8235, *Pseudomonas sp.* DSM 8653, *Variovorax paradoxus* (= *Alcaligenes paradoxus*) DSM 8244.
